(19) Europäisches Patentamt
European Patent Office
Office européen des brevets

(11) Publication number: **0 291 130 A2**

# (12) EUROPEAN PATENT APPLICATION

(21) Application number: 88200926.9

(22) Date of filing: 10.05.88

(51) Int. Cl.[4]: **C12M 1/40 , C12N 11/14 , G01N 27/40 , //C12Q1/54**

(30) Priority: **15.05.87 IT 2052987**

(43) Date of publication of application: **17.11.88 Bulletin 88/46**

(84) Designated Contracting States: **AT BE CH DE ES FR GB GR LI LU NL SE**

(71) Applicant: **ENIRICERCHE S.p.A.**
**Corso Venezia 16**
**I-20121 Milan(IT)**

(72) Inventor: **Colapicchioni, Claudio**
**Via San Castulo 3**
**I-00182 Rome(IT)**
Inventor: **Barbaro, Aurelio**
**Via Madonna 2 Ponti**
**I-00060 Capena Rome(IT)**
Inventor: **Baroncelli, Vittorio**
**Via Tagliamento 20**
**I-00198 Rome(IT)**
Inventor: **Battilotti, Massimo**
**Via Romolo Gigliozzi 53**
**I-00128 Tor de Cenci Rome(IT)**

(74) Representative: **Roggero, Sergio et al**
**Ing. Barzanò & Zanardo Milano S.p.A. Via Borgonuovo 10**
**I-20121 Milano(IT)**

## (54) Biosensor with enzymatic membrane chemically bound to a semiconductor device.

(57) A biosensor is disclosed, together with the relevant process for preparing it, which is constituted by an enzymatic membrane, a device of EOS or CHEMFET type containing at its surface silicon oxide adhering to said membrane by means of a polysiloxanic matrix, characterized in that the enzyme is chemically bound to the polysiloxanic matrix either by means of bifunctional coupling agents (possibly by polymerizing the same enzyme together with support biopolymers), or by means of diazo groups, and that the polysiloxanic matrix is selected from functional organosilanes of general formula

$$YR - \underset{R^{IV}}{\overset{R^{II}}{Si}} - R^{III} \qquad (1)$$

wherein:

$R^{II}$, $R^{III}$, $R^{IV}$, which may be either equal to, or different from, one another, are alkyl or alkoxy groups containing from 1 to 10 carbon atoms,

$R = (CH_2)_m X (CH_2)_n$

EP 0 291 130 A2

wherein X is $CH_2$, or an either mono-or polycondensed aromatic group, or NH or O,

m and n, which may be either equal to, or different from, one another, are integers which can have a value comprised within the range of from 0 to 10, with value 0 being excluded when X is either NH or O,

Y can be $-NH_2$ or -OH or -SH,

or from the functional organosilanes of general formula

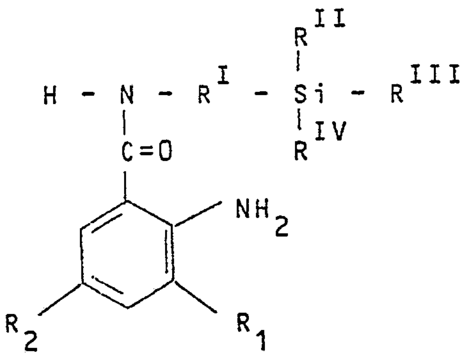

(2)

wherein:

$R_1$, $R_2$, which may be either equal to, or different from, one another, are Cl, Br, $CH_3$, $NO_2$, $NH_2$ or H,

$R^{II}$, $R^{III}$, $R^{IV}$, which may be either equal to, or different from, one another, are alkyl or alkoxy groups containing from 1 to 10 carbon atoms,

$R^I$ can be an alkyl, aminoalkyl, aminoalkylaryl, alkylaryl group containing from 1 to 10 carbon atoms.

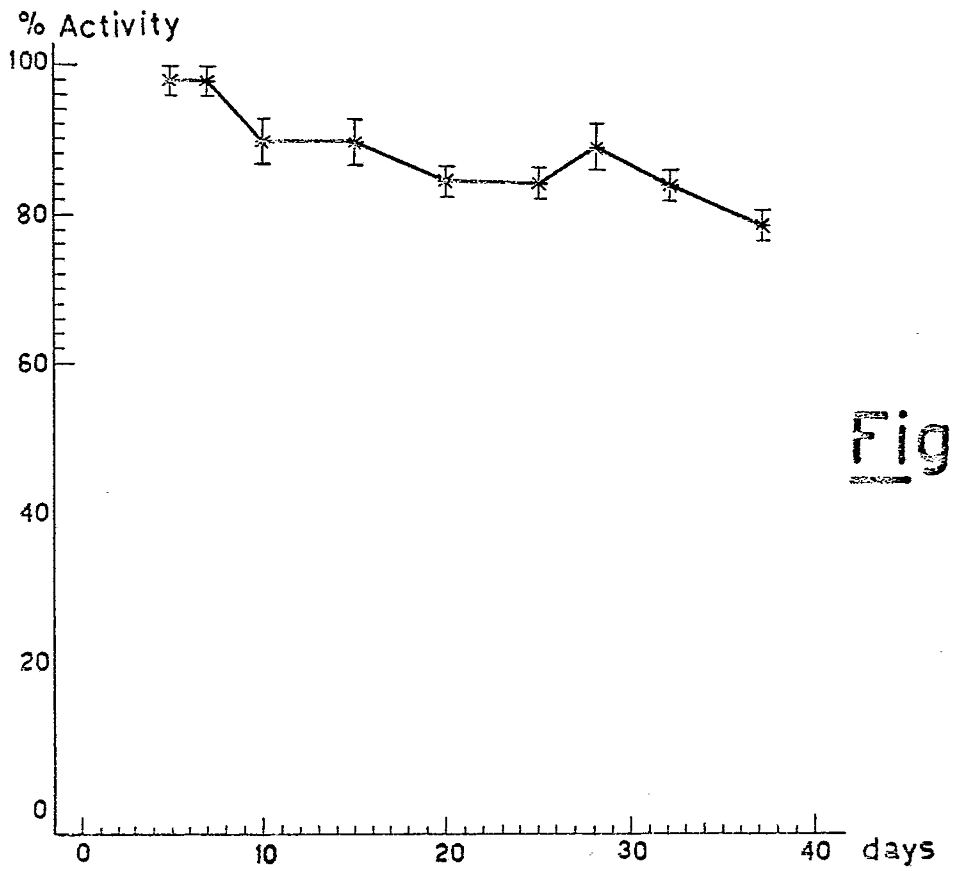


Fig.1

# "BIOSENSOR WITH ENZYMATIC MEMBRANE CHEMICALLY BOUND TO A SEMICONDUCTOR DEVICE"

The present invention relates to the realization of an enzyme device responding to the substrate of the same enzyme, together with the relevant processes of preparation, which contains an enzymatic membrane constituted by a mono-layer of the enzyme alone, or by a polymeric multi-layer constituted by the enzyme bound together with support biopolymers, a polysiloxanic matrix and a device of EOS (Electrolyte oxide semiconductor) or CHEMFET type.

In the realization of enzyme-sensors which use semiconductor devices (either EOS or CHEMFET), the enzyme is generally embedded in a gel of polyacrylamide, albumin or of any other types, and is made only physically adhere to the silicon oxide of the same device.

Several types of devices are known, wherein the enzyme-containing membrane is only physically deposited.

The major drawback of such devices consists in that water can penetrate the interface between the membrane and silicon oxide, and cause the same membrane to come off. However, the enzymatic activity of enzymes physically immobilized on devices of either EOS or CHEMFET type does not generally last for longer than 2 weeks.

In order to prevent the membrane from coming off, devices have been realized recently, and are disclosed in Japanese patent applications (to Kokai) Nos. 59/203951, 60/247151 and 60/251883, wherein the enzymatic membrane is chemically bound to the silane present in the device.

However, such devices show a relatively short stability, i.e., not longer than one month. The present Applicant has surprisingly found that, by realizing a device with an enzymatic membrane chemically bound by means of bifunctional coupling agents, or by means of diazo groups, to a polysiloxanic matrix selected from determined organosilanes, the stability of the enzyme is retained for a longer period, with furthermore the possibility being avoided that the enzymatic membrane may come off from the polysiloxanic layer.

A first object of the present invention is a biosensor containing an enzymatic membrane, a device of EOS or CHEMFET type containing at its surface silicon oxide adhering to said enzymatic membrane by means of a polysiloxanic matrix, characterized in that the enzyme is chemically bound to the polysiloxanic matrix either by means of bifunctional coupling agents which bind the amino groups present in the same polysiloxanic matrix, with the same enzyme being possibly polymerized together with support biopolymers, or by means of diazo groups formed in the same polysiloxanic matrix, and that the polysiloxanic matrix is selected from functional organosilanes of general formula

$$\begin{array}{ccccc} & & R^{II} & & \\ & & | & & \\ YR & - & Si & - & R^{III} \\ & & | & & \\ & & R^{IV} & & \end{array} \qquad (1)$$

wherein:

$R^{II}$, $R^{III}$, $R^{IV}$, which may be either equal to, or different from, one another, are alkyl or alkoxy groups containing from 1 to 10 carbon atoms,

$R = (CH_2)_m X (CH_2)_n$

wherein X is $CH_2$, or an either mono-or polycondensed aromatic group, or NH or O,

$\underline{m}$ and $\underline{n}$, which may be either equal to, or different from, one another, are integers which can have a value comprised within the range of from 0 to 10, with value 0 being excluded when X is either NH or O,

Y can be $-NH_2$ or -OH or -SH,

or from the functional organosilanes of general formula

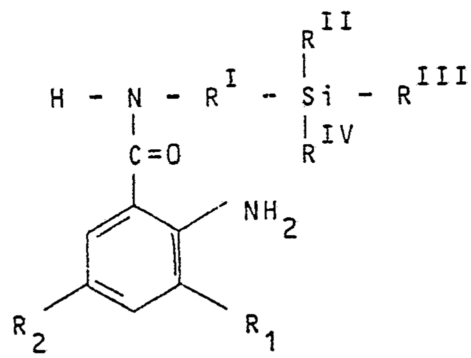

(2)

wherein

$R_1$, $R_2$, which may be either equal to, or different from, one another, are Cl, Br, $CH_3$ , $NO_2$, $NH_2$ or H,

$R^{II}$, $R^{III}$, $R^{IV}$, which may be either equal to, or different from, one another, are alkyl or alkoxy groups containing from 1 to 10 carbon atoms,

$R_I$ can be an alkyl, aminoalkyl, aminoalkylaryl, alkylaryl group containing from 1 to 10 carbon atoms.

For exemplifying purposes, as the polysiloxanic matrix, aminoethyl-aminopropyl-trimethoxysilane (AEAPS) or anthranylamide-propyl-triethoxysilane (AAPS) can be used.

The device of EOS type, besides silicon oxide at its surface, should contain, in its portion under the same silicon oxide, a layer of aluminum or gold deposited by evaporation.

In case a functional organosilane is used, which has the above specified formula (2), the process for obtaining it is based on the reaction between isatoic anhydride or derivatives thereof, and aminosilanes of

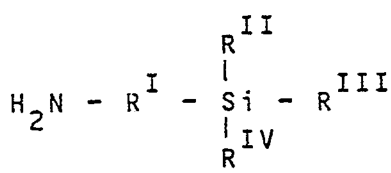

type, according to the following reaction diagram:

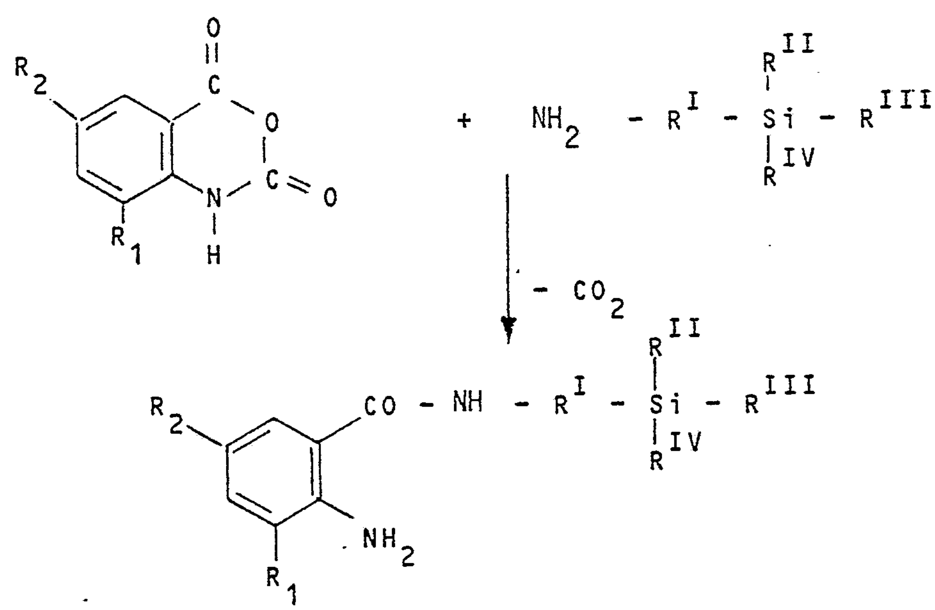


The practically preferred silanes for obtaining the functional organosilane according to the purposes of the present invention are:

- 3-aminopropyl-triethoxysilane

$H_2N-(CH_2)_3-Si-(OC_2H_5)_3$ )3
- aminomethyl-triethoxysilane
$H_2N-CH_2-Si-(OC_2H_5)_3$
- 2-aminoethyl-aminopropyl-trimethoxysilane
$H_2N-(CH_2)_2-NH-(CH_2)_3-Si-(OCH_3)_3$
- 2-aminoethyl-aminopropyl-triethoxysilane
$H_2N-(CH_2)_2-NH-(CH_2)_3-Si-(OC_2H_5)_3$
- 2-aminoethyl-aminopropyl-methyl-dimethoxysilane

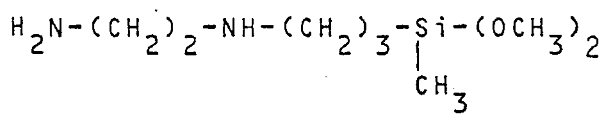

The synthesis reaction is carried out by slowly adding isatoic anhydride to the aminosilane in the presence of, or without, a solvent. When the reaction is carried out in a solvent, it is obviously necessary that this latter does not react with isatoic anhydride; in case of hydroxy-containing solvents, the reactions should be carried out, e.g, under mild reaction conditions, and in the absence of possible catalysts.

However, as solvents alcohols, ethers, chlorinated solvents, etc., can be used; obviously, the reactions should be carried out in the absence of water, which would lead to the hydrolysis of the alkoxy groups.

The reaction can be also carried out by adding the isatoic anhydride all at a once, reminding that the reaction is slightly exothermal (cooling will be necessary) and that, notwithstanding the considerable difference in reactivity between the aliphatic amine (the aminosilane) and the aromatic amine (the reaction product), oligomeric products of

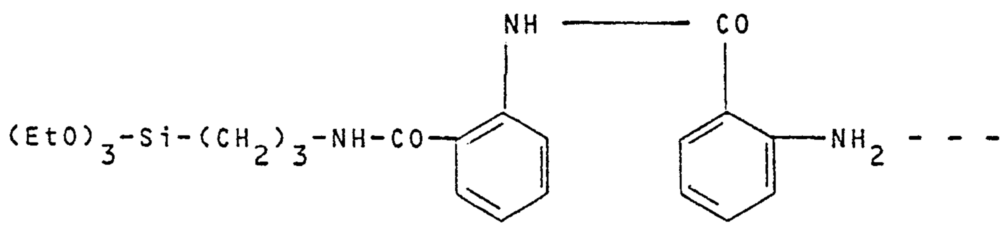

type can arise, even if in any case very small amounts thereof will be formed.

Into said device, enzyme types can be introduced, which, during the catalysed reaction, produce electrical charges.

The support biopolymers can be proteins (e.g., albumin), biopolymers having functional groups (e.g., polylysine), or functionalized biocompatible polymers (e.g., polyvinyl alcohol).

Another object of the present invention consists in the process for obtaining the enzyme-device.

A first process comprises the following process steps:

-preparation of a siloxanic prepolymer, followed by one or more deposition(s) of same siloxanic prepolymer on a device of either EOS or CHEMFET type, and by a thermal curing, during which the complete polymerization takes place by hydrolysis of the alkoxy groups of the silane, with a polysiloxanic matrix, and the consequent chemical bonding of said matrix to silicon oxide being obtained owing to the reaction of further alkoxy groups with the Si-OH hydroxy groups existing on the oxidated surface;

-activation of the aliphatic amino groups present on the polysiloxanic layer with bifunctional coupling agents;

-addition of the enzyme in a solution buffered at a pH value comprised within the range of from 6.5 to 7.5 to the activated silane layer.

The above disclosed process makes it possible monolayer enzimatic membranes to be obtained, wherein the enzyme is chemically bonded to the surface of the EOS or CHEMFET device by means of the polysiloxanic layer.

In case the enzyme is chemically bonded both to a support biopolymer, so as to favour its stability, and to the surface of EOS or CHEMFET device, by means of the polysiloxanic layer, with multy-layer enzymatic membranes being obtained, the process comprises the following process steps:

-preparation of a siloxanic prepolymer, followed by one or more deposition(s) of same siloxanic

prepolymer on a device of either EOS or CHEMFET type, and by a thermal curing, during which the complete polymerization takes place by hydrolysis of the alkoxy groups of the silane, with a polysiloxanic matrix, and the consequent chemical bonding of said matrix to silicon oxide being obtained owing to the reaction of further alkoxy groups with the Si-OH hydroxy groups existing on the oxidated surface;

-activatiôn of the aliphatic amino groups present on the polysiloxanic layer with bifunctional coupling agents;

-preparation of an enzymatic prepolymer by reacting the enzyme and a support biopolymer by means of bifunctional coupling agents;

-reaction of the above disclosed enzymatic prepolymer with the activated amino groups of the polysiloxanic layer.

The bifunctional coupling agents used in both above reported processes can be selected from dialdehydes (e.g., glutaraldehyde), or from diisocyanates (e.g., 2,4-toluene-diisocyanate).

The support biopolymers can be selected from proteins (e.g., albumin), biopolymers containing functional groups (e.g., polylysine), or functionalized biocompatible polymers (e.g., polyvinyl-alcohol).

A third process makes it possible an enzymatic membrane to be obtained, which is constituted by a monolayer of an enzyme chemically bonded by means of diazo groups to the polysiloxanic layer obtained from a silane, wherein a free aromatic amino group is present (e.g., AAPS = anthranylamide-propyl-triethoxysilane).

The advantage of this process is in this case the rapidity with which the chemical bond between the enzyme and the polysiloxanic layer can be realized

The presence of the aromatic ring in the polysiloxanic layer enhances the hydrophobic characteristics of this latter, rendering more difficult the water penetration into the interface between the surface silicon oxide and the polysiloxanic matrix.

Furthermore, such a type of enzymatic immobilization result advtantageous in all those cases in which the bond with the aliphatic amine of the enzymatic lysine, often used in the enzymatic immobilizations, is near the active site, and causes a decrease in enzymatic activity. Said process comprises the following process steps:

-preparation of a siloxanic prepolymer wherein the monomer silane contains a free aromatic amine followed by one or more deposition(s) of same siloxanic prepolymer on a device of either EOS or CHEMFET type, and by a thermal curing, during which the complete polymerization takes place by hydrolysis of the alkoxy groups of the silane, with a polysiloxanic matrix, and the consequent chemical bonding of said matrix to silicon oxide being obtained owing to the reaction of further alkoxy groups with the Si-OH hydroxy groups existing on the oxidated surface;

-formation of the diazo group of the aromatic amine of the silane with nitrous acid at a temperature of approximately 5° C and cold washing up to neutral pH;

-formation of the bond between the diazo group and the tyrosine of the enzyme at a temperature of approximately 5° C, in 0.02 M phosphate buffer, pH 7.

Inasmuch as nitrous acid is unstable, it is formed "in situ", by adding sodium nitrite to a solution of hydrochloric acid.

The deposition of the siloxanic prepolymer in the above cited processes is carried out by means of techniques, denominated as "spin-on techniques", and the activation of the polysiloxanic layer and the reaction of this latter with the pre-polymerized enzymatic solution are carried out on a swinging plate.

The thermal treatments of the siloxanic pre-polymer are carried at temperatures comprised within the range of from 80 to 140° C, and the subsequent treatments of enzymatic immobilization are carried out at a temperature comprised within the range of from 25 to 37° C.

The thickness of deposited siloxanic prepolymer should be comprised within the range of from 0.5 to 3$\mu$, whilst the enzymatic membrane can have a thickness comprised within the range of from 0.5 to 2$\mu$.

In order to realize the polysiloxanic layer, the rotary-disk equipment used for the deposition(s) should preferably operate at a revolution speed comprised within the range of from 400 to 4,500 rpm.

Some examples are given now in order to better illustrate the invention, without however limiting it.

## Example 1

An enzyme-device was realized according to the process which makes it possible a multi-layer enzymatic membrane to be obtained (with the enzyme being polymerized together with support biopolymers) chemically bonded to the polysiloxanic matrix.

On small test pieces of 1 cm x 1 cm of dimensions of an EOS device, a solution of a polysiloxane

partially hydrolysed in a water-alcoholic medium was deposited, which contained:
- 21% of aminoethyl-aminopropyl-trimethoxysilane (AEAPS);
- 3% of $CH_3COOH$;
- 1% of $H_2O$
in absolute ethanol.

Three depositions were carried out by means of a rotary-disk apparatus running at a revolution speed of 3,500 rpm for 20 seconds.

The excess of this solution of oligomer deposited on MOS was centrifuged off by means of the rotary motion of the bearing plate. After thermal curing for 16 hours at 120°C, the test pieces were let cool, were placed in a weighing bottle in a solution of 0.02 M phosphate buffer, pH 7, containing a concentration of 1% of glutaraldehyde and were stirred on a shaking plate for 1.5 hours at room temperature, and were finally washed with the same phosphate buffer.

10 mg of glucose-oxidase and 5 mg of bovine seralbumin (BSA) were separately reacted in 2 ml of 0.02 M phosphate buffer at pH 7 for 30 minutes at room temperature, in the presence of 0.5% glutaraldehyde, in order to produce an initial polymerization of the enzyme with the protein. This solution was then added to the bars, wherein the polysiloxanic layer was activated with glutaraldehyde, together with 1 ml of 0.02 M phosphate buffer and let react for 1.5 hours at room temperature; the devices were then washed with the same buffer solution.

In Figure 1, the activity (as %) is shown (on the ordinate) as a function of time (as days) (on the abscissa) of samples wherein the enzyme (glucose-oxidase) was immobilized on the polysiloxanic layer together with bovine seralbumin (BSA), by the above disclosed methods. The activity was measured by means of kits available on the market (by BOEHRINGER), by determining the decrease in substrate (glucose).

One can observe that the activity of the enzymatic membrane, immobilized on EOS devices, maintained inside a buffer solution at pH 7, is retained after one month by more than 80%.

<u>Example 2</u>

An enzyme-device was realized according to the process which makes it possible a multi-layer enzymatic membrane to be obtained (with the enzyme being polymerized together with support biopolymers) chemically bonded to the polysiloxanic matrix.

The process is analogous to that of Example 1, with the difference that instead of glucose-oxidase and bovine seralbumin (BSA), 10 mg of urease and 10 mg of polylysine are separately reacted.

In Figure 2, the activity (as %) is shown (on the ordinate) as a function of time (as days) (on the abscissa) of samples wherein the urease enzyme was immobilized on the polysiloxanic layer together with polylysine by means of the above disclosed methods. The activity was measured by means of kits available on the market (by BOEHRINGER), by determining the formation of the reaction product (urea).

One can observe that the activity of the enzymatic membrane, immobilized on EOS devices, maintained inside a buffer solution at pH 7, is retained after one month by more than 80%.

In Figure 3, the curves are reported, which show the trend of the capacity (as nF) as a function of the voltage (as volts) at 15 Hz of the EOS device on which an enzymatic membrane was immobilized, which is constituted by urease together with polylysine according to the above disclosed methods.

The capacity/voltage tests are generally used in semiconductor technology in order to study the characteristics of silicon oxide when field-effect transistors are manufactured.

When the enzyme is immobilized on EOS devices, during the enzymatic activity charges are formed on the device surface, which cause a shift of its capacity minimum.

From Figure 3, such a shift can be observed when a $10^{-3}$ M solution of potassium chloride ("a" curve) is replaced by a 0.1 M solution of urea in $10^{-3}$ M potassium chloride ("b" curve). Then, when the $10^{-3}$ M solution of potassium chloride is restored, the minimum of capacity return to its initial values ("c" curve).

Furthermore, from Figure 4 (wherein the charts are reported, with show the capacity (as nF) as a function of the voltage (as volts) at 15 Hz of an EOS device - on which a polysiloxanic layer of AEAPS was deposited, in the presence of 0.1 M urea in $10^{-3}$ KCL ("a curve") - and of the same EOS device, in the absence of urea ("b" curve), one can see that urea alone does not cause a shift in capacity/voltage curve.

The shift of the minimum of capacity, observed in Figure 3, can therefore be undoubtedly attributed to the enzymatic activity, which causes electrical charges to form at silicon/silicon oxide interface.

Example 3

An enzyme-device was realized according to the process which makes it possible a mono-layer enzymatic membrane of glucose-oxidase chemically bonded to the polysiloxanic matrix to be formed, by starting from a monomer silane wherein an aromatic amine was present, by means of diazo groups.

On small test pieces of 1 cm x 1 cm of an EOS device, a solution of a polysiloxane partially hydrolysed in a water-alcoholic vehicle was deposited, which contained:

- 21% of anthranylamide-propyl-triethoxysilane;
- 4% of acetic acid;
- 1% of $H_2O$

in absolute ethanol.

Three depositions were carried out by means of a rotary-disk apparatus running at a revolution speed of 3,500 rpm for 20 seconds, followed by a thermal curing at 120° C for 16 hours.

The so silanized devices were charged to a reactor with jacket, kept at the controlled temperature of +5° C. Then, 30 ml of 2 N HCl were added, and finally a solution of 1.8 g of $NaNO_2$ in 10 ml was added dropwise.

After one hour from the addition of $NaNO_2$, the devices were washed with neutral water, to neutral pH.

Then, to the reactor 40 ml of a solution were added, which contained 0.5 mg/ml of glucose-oxidase in a solution of 0.02 M phosphate buffer, pH 7, the reaction was let proceed for 2 hours at 5° C with stirring, and washes were carried out with the same phosphate buffer solution.

In Figure 5, the activity (as %) is shown (on the ordinate) as a function of time (as days) (on the abscissa) of samples wherein the enzyme was immobilized by means of the above-disclosed process. One can observe that the activity of the samples, stored in a buffer solution at pH 7, remains good for at least one month.

In Figure 6, the curves are reported, which show the trend of the capacity (as nF) as a function of the voltage (as volts), measured at 1 Hz, of an EOS device on which the glucose-oxidase enzyme was immobilized by means of diazo groups, according to the above disclosed techniques.

In this case too, the shift of capacity minimum can be observed, which occurs when a $10^{-3}$ M solution of potassium chloride ("a" curve) is replaced by a 0.1 M solution of glucose in $10^{-3}$ M potassium chloride ("b" curve), and is due to the production of charges during the enzymatic activity.

In Figures 7, 8 and 9, the Fourier Transform I.R. spectra (F.T.I.R.) are represented, which are respectively obtained under reflectance on an EOS device on which a polysiloxanic layer of AAPS was deposited, after glucose-oxidase immobilization by means of diazo groups (Figure 7), in accordance with the invention, after treatment with nitrous acid alone (Figure 8), and with no treatments (Figure 9).

In the spectrum relevant to the immobilized enzyme, the presence can be observed of a band at 3,400 $cm^{-1}$, which is due to the stretching of NH of the amidic bond, and does not appear in the spectrum of the sample treated with nitrous acid only.

Example 4

A glucose-selective CHEMFET device was prepared, which contained an enzymatic membrane chemically bonded to an AEAPS polysiloxanic layer deposited on the gate of a FET, realized according to as disclosed in Example 1.

On a FET device without contacts, containing a membrane gate and a reference gate, of 2.3 mm x 3.1 mm of dimensions, the following operations were made:

-Mechanical masking of the area of the contacts provided on the device, with a suitable special, heat-resistant adhesive tape;

-Deposition on the gate of a solution containing an organosilane, partially hydrolysed in a water-alcoholic medium constituted by:

- 21% aminoethyl-aminopropyl-trimethoxysilane;
- 3% acetic acid;
- 1% water,

in absolute ethanol.

The deposition was carried out by means of a rotary-disk equipment revolving at 5,000 rpm for 30 seconds (spin-coating). The device underwent then a thermal curing at 120° C for a time of 16 hours, during

which ' the complete polymerization occurred by hydrolysis of the alkoxy groups of silane, with a polysiloxanic matrix, and the consequent chemical bonding of said matrix to silicon oxide being obtained, thanks to the reaction of further alkoxy groups with Si-OH hydroxy groups present on FET surface.

-Mechanical removal of the adhesive layer used as the mask for the deposition of the polysiloxanic layer;

-Housing of the device on a commercial metal support of TO5 type;

-Application of the contacts of the device on the support, by using an ultra-sound microwelder;

-Masking of the area wherein the polysiloxane layer is present;

-Deposition of a suitable encapsulating material on the device provided with the contacts, and on the support;

-Mechanical removal of the masking material present on the polysiloxanic layer;

-Realization of glucose-oxidase-containing enzymatic membrane according to as disclosed in Example 1.

In Figure 10, the response of the above-disclosed CHEMFET device to glucose in 20 mM phosphate buffer pH 7 is reported. On the abscissa, the glucose concentration logarithm, and on the ordinate the change in threshold response as mV is reported.

Example 5

An enzyme-device was realized according to the process which makes it possible a multi-layer membrane chemically bonded to the polysiloxanic matrix to be obtained. The process is analogous to that as of Example 1, with the difference that in lieu of glucose-oxidase, acetylcolinesterase is used.

The device can be used for the determination of pesticides (organic phosphates or carbamates) which are known to inhibit the activity of acetylcolinesterase (e.g.: R.D: O'Brien, Insecticides: Action and Metabolism, Academic Press, New York 1967, pages 39-54, 86-95; I.R. Corbett, The Biochemical Mode of Action of Pesticides, Academic Press, New York 1974, page 540).

Various methods for measuring pesticides, based on acetylcolinesterase inhibition, as radiometric, colorimetric or fluorimetric methods have been developed in the past (M.H. Sadar, S.S. Kuan and G.G. Guilbault, Analytical Chemistry 42, 1770 (1970)).

The advantage of a system based on FET devices is represented by a low cost, easy use, small amounts of enzyme used.

On small test pieces of 1 cm x 1 cm of an EOS device, a polysiloxanic layer was deposied according to as already disclosed in Example 1. After thermal curing for 16 hours at 120°C, the test pieces were let cool, were placed in a weighing bottle, and were treated with glutaraldehyde as hereinabove disclosed.

0.5 mg of acetylcolinesterase and 0.5 mg of bovine seralbumin (BSA) were separately reacted in 0.5 ml of 0.02 M phosphate buffer at pH 7 for 30 minutes at room temperature, in the presence of 0.05% glutaraldehyde. This solution was then reacted with the test pieces treated with glutaraldehyde, as disclosed in Example 1.

In Figure 11 the inhibition of the activity of acetylcolinesterase (immobilized on EOS devices) vs. various concentration of Malathion pesticide is reported. Curve 1 represents the activity of the enzyme as such, curve 2 represents the activity of the enzyme after the addition of 33 µg/litre of Malathion, curve 3 represents the activity of the enzyme after the addition of 95 µg/litre of Malathion, curve 4 represents the activity of the enzyme after the addition of 208 µg/litre of Malathion, and finally curve 5 shows the nearly total restoration of the activity of the enzyme after that the pesticide is washed off. The enzymatic activity was determined by spectrophotometry with a commercial kit by BOEHRINGER.

**Claims**

1. Biosensor containing an enzymatic membrane, a device of EOS or CHEMFET type containing at its surface silicon oxide adhering to said enzymatic membrane by means of a polysiloxanic matrix, characterized in that the enzyme is chemically bound to the polysiloxanic matrix either by means of bifunctional coupling agents, with the same enzyme being possibly polymerized together with support biopolymers, or by means of diazo groups formed in the same polysiloxanic matrix, and that the polysiloxanic matrix is selected from functional organosilanes of general formula

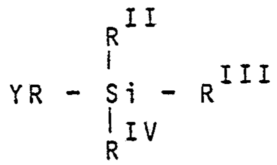

(1)

wherein:

$R^{II}$, $R^{III}$, $R^{IV}$, which may be either equal to, or different from, one another, are alkyl or alkoxy groups containing from 1 to 10 carbon atoms,

R = $(CH_2)_m$ X $(CH_2)_n$ wherein X is $CH_2$, or an either mono-or polycondensed aromatic group, or NH or O,

$_m$ and $_n$, which may be either equal to, or different from, one another, are integers which can have a value comprised within the range of from 0 to 10, with value 0 being excluded when X is either NH or O,

Y can be $-NH_2$ or -OH or -SH, or from the functional organosilanes of general formula

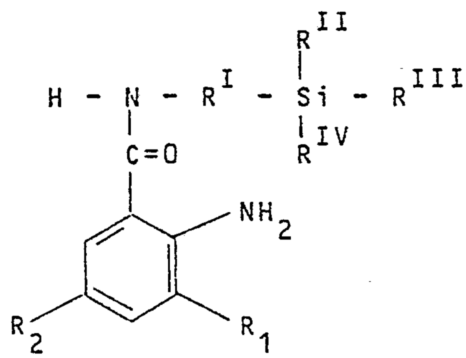

(2)

wherein

$R_1$, $R_2$, which may be either equal to, or different from, one another, are Cl, Br, $CH_3$, $NO_2$, $NH_2$ or H,

$R^{II}$, $R^{III}$, $R^{IV}$, which may be either equal to, or different from, one another, are alkyl or alkoxy groups containing from 1 to 10 carbon atoms,

$R^{I}$ can be an alkyl, aminoalkyl, aminoalkylaryl, or an alkylaryl group containing from 1 to 10 carbon atoms.

2. Process for obtaining a biosensor according to claim 1, which comprises the following process steps:

-preparation of a siloxanic prepolymer, followed by one or more deposition(s) of the same siloxanic prepolymer on a device of either EOS or CHEMFET type, and by a thermal curing, during which the complete polymerization takes place by hydrolysis of the alkoxy groups of the silane, with a polysiloxanic matrix, and the consequent chemical bonding of said matrix to the silicon oxide being obtained owing to the reaction of further alkoxy groups with the Si-OH hydroxy groups present on the oxidated surface;

-activation of the aliphatic amino groups present on the polysiloxanic layer with bifunctional coupling agents;

-addition of the enzyme in a solution buffered at a pH value comprised within the range of from 6.5 to 7.5 to the activated silane layer,

with the depositions of the siloxanic prepolymer being carried out by means of a rotary-disk equipment.

3. Process for obtaining a biosensor according to claim 1, which comprises the following process steps:

-preparation of a siloxanic prepolymer, followed by one or more deposition(s) of same siloxanic prepolymer on a device of either EOS or CHEMFET type, and by a thermal curing, during which the complete polymerization takes place by hydrolysis of the alkoxy groups of the silane, with a polysiloxanic matrix, and the consequent chemical bonding of said matrix to silicon oxide being obtained owing to the reaction of further alkoxy groups with the Si-OH hydroxy groups existing on the oxidated surface;

-activation of the aliphatic amino groups present on the polysiloxanic layer with bifunctional coupling agents;

-preparation of an enzymatic prepolymer by reacting the enzyme and a support biopolymer by means of bifunctional coupling agents;

-reaction of the above disclosed enzymatic prepolymer with the activated amino groups of the polysiloxanic layer,
with the depositions of the siloxanic prepolymer being carried out by means of a rotary-disk equipment.

4. Process for obtaining a biosensor according to claim 1, which comprises the following process steps:

-preparation of a siloxanic prepolymer wherein the monomer silane contains a free aromatic amine, followed by one or more deposition(s) of same siloxanic prepolymer on a device of either EOS or CHEMFET.type, and by a thermal curing, during which the complete polymerization takes place by hydrolysis of the alkoxy groups of the silane, with a polysiloxanic matrix, and the consequent chemical bonding of said matrix to silicon oxide being obtained, owing to the reaction of further alkoxy groups with the Si-OH hydroxy groups existing on the oxidated surface;

-formation of the diazo group of the aromatic amine of the silane with nitrous acid at a temperature of approximately 5 °C and cold washing up to neutral pH;

-formation of the bond between the diazo group and the tyrosine of the enzyme at a temperature of approximately 5 °C, in 0.02 M phosphate buffer, pH 7,
with the deposition(s) of the siloxanic prepolymer being carried out by means of a rotary-disk equipment.

5. Process according to claim 2 or 3 or 4, wherein the preliminary thermal curing of the silanic prepolymer is carried at a temperature comprised within the range of from 80 to 140 °C.

6. Process according to claim 2 or 3 or 4, wherein the deposition(s) of siloxanic prepolymer are carried out in such a way as to form a thickness comprised within the range of from 0.5 to 3$\mu$.

7. Process according to claim 2 or 3 or 4, wherein the rotary-disk equipment used for the deposition(s) operates at a revolution speed comprised within the range of from 400 to 4,500 rpm.

8. Process according to claims 1 or 2 or 3, wherein the bifunctional coupling agents are selected from dialdehydes.

9. Process according to claim 8, wherein the dialdehyde is glutaraldehyde.

10. Process according to claims 1 or 2 or 3, wherein the bifunctional coupling agents are selected from diisocyanates.

11. Process according to claim 10, wherein the diisocyanate is 2,4-toluylene-diisocyanate.

12. Process according to claims 1 and 3, wherein the support biopolymers are selected from proteins, or from biopolymers containing functional groups, or from functionalized compatible biopolymers.

13. Process according to claim 12, wherein the protein is albumin.

14. Process according to claim 12, wherein the biopolymer containing functional groups is polylysine.

15. Process according to claim 12, wherein the functionalized compatible biopolymer is polyvinylalcohol.

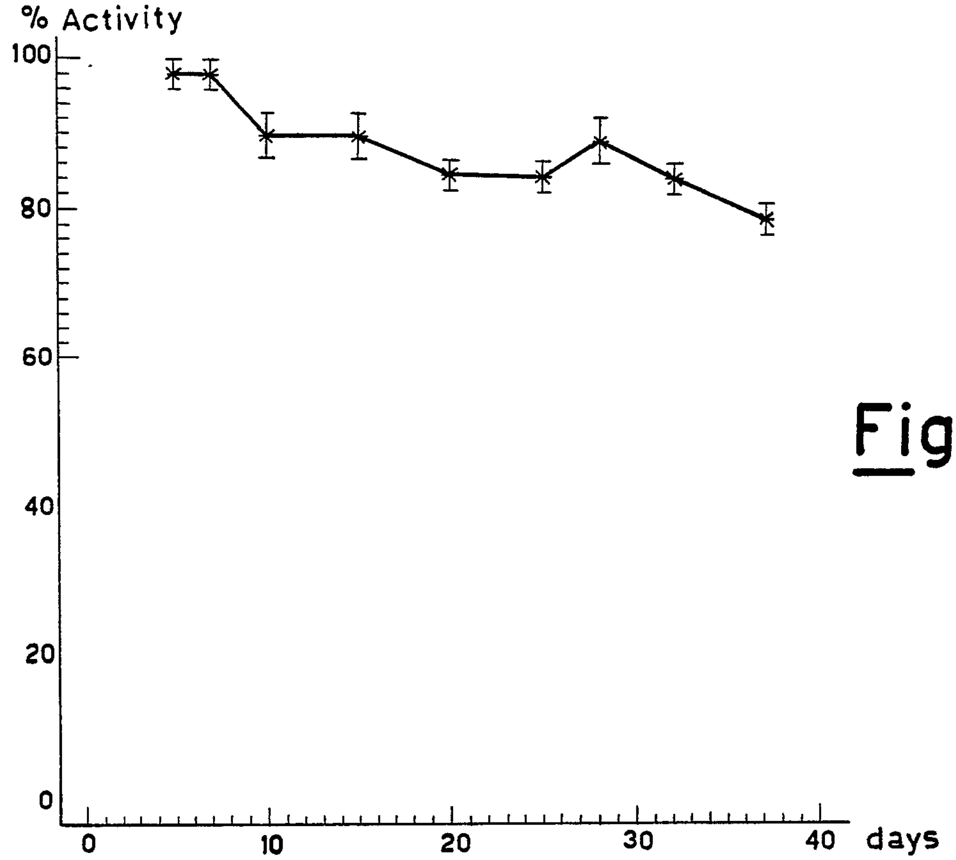
% Activity
100
80
60
40
20
0
0
10
20
30
40
days

Fig.1

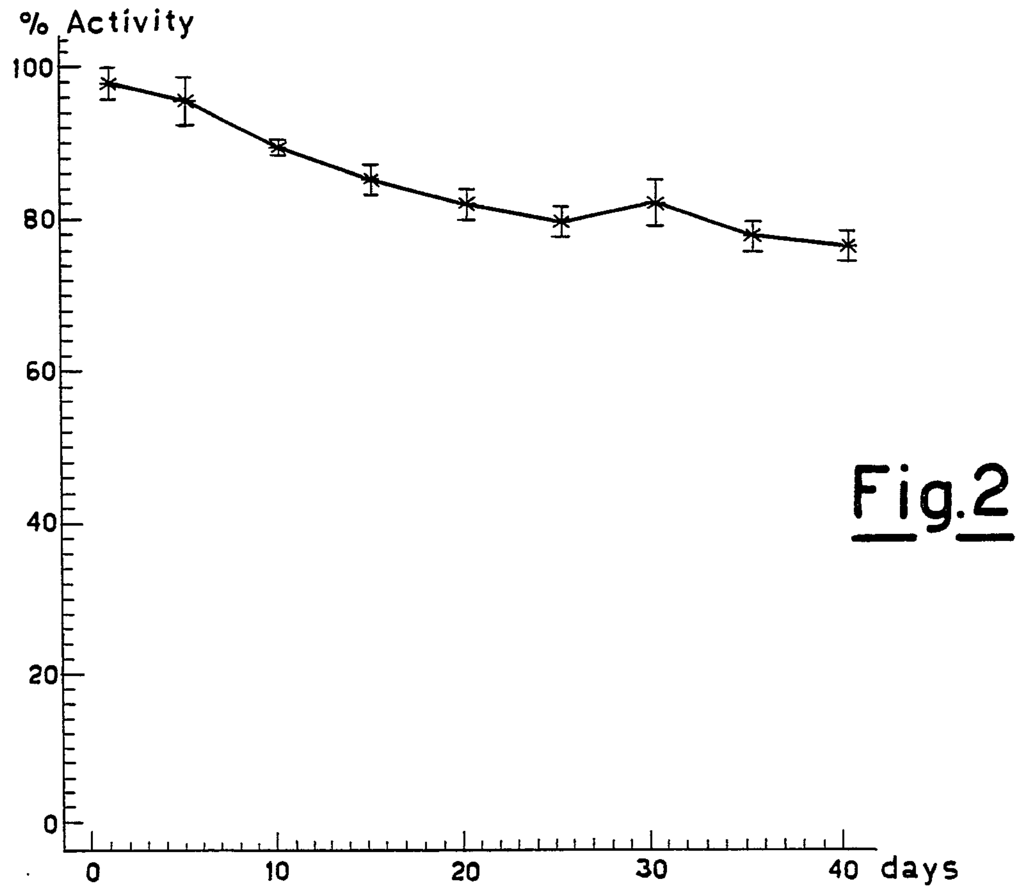
% Activity
100
80
60
40
20
0
0
10
20
30
40
days

Fig.2

Fig.3

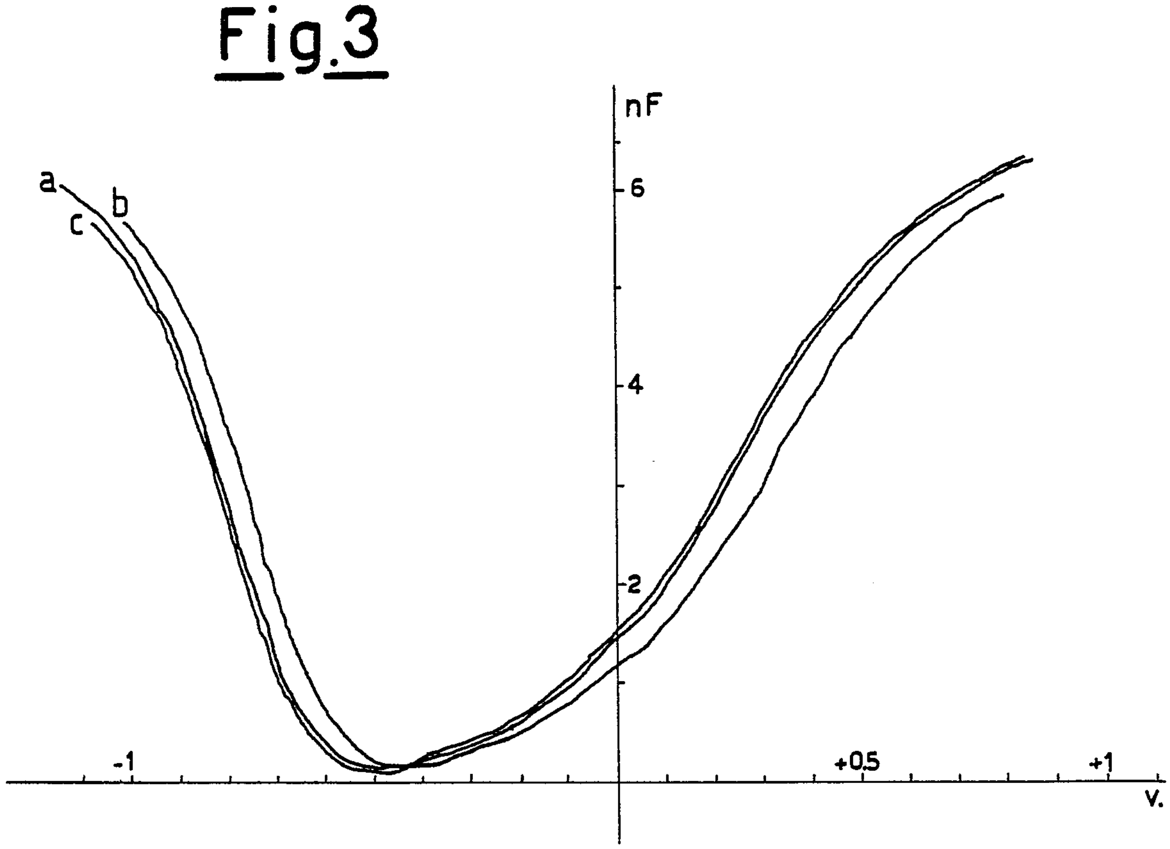


Fig.4

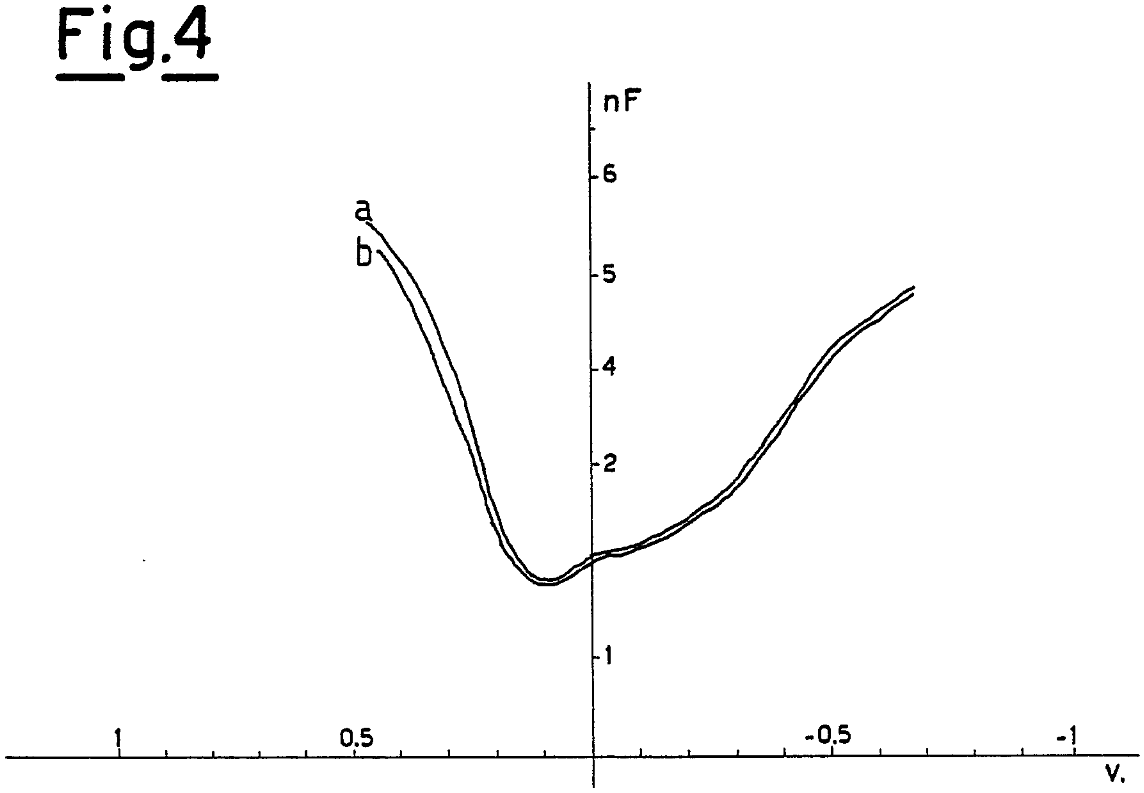

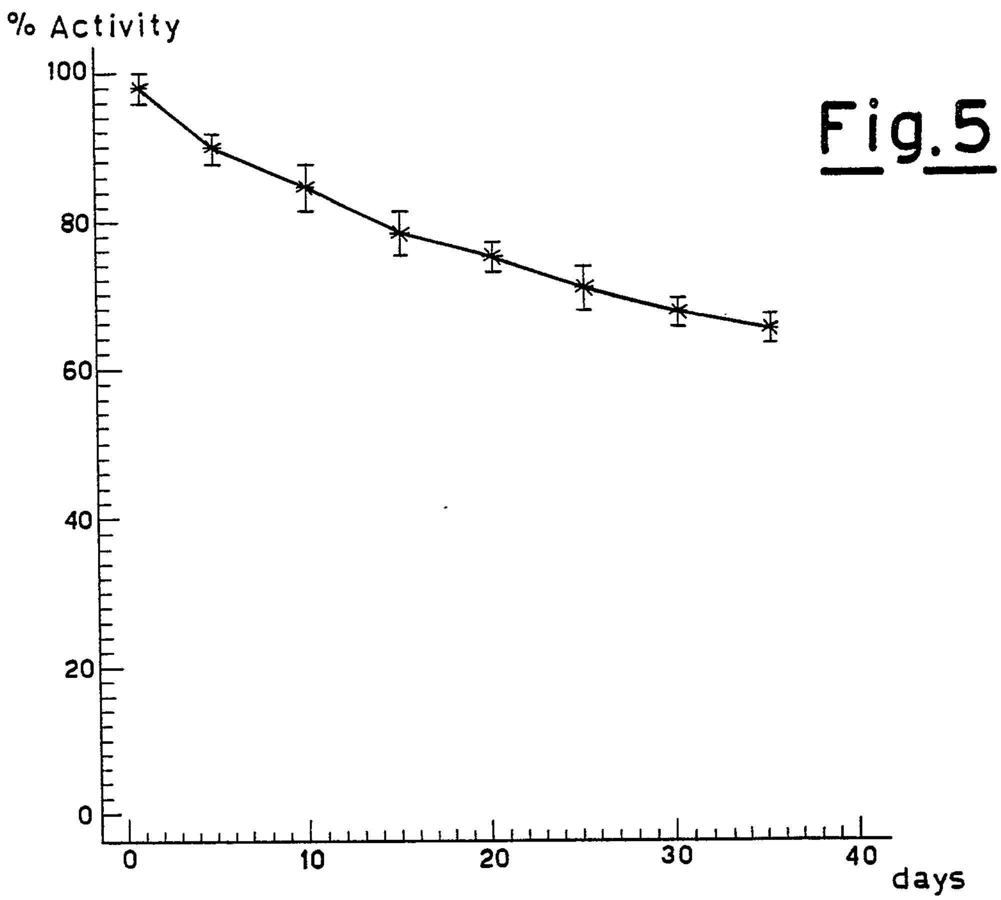
% Activity
100
80
60
40
20
0
0
10
20
30
40
days

Fig.5

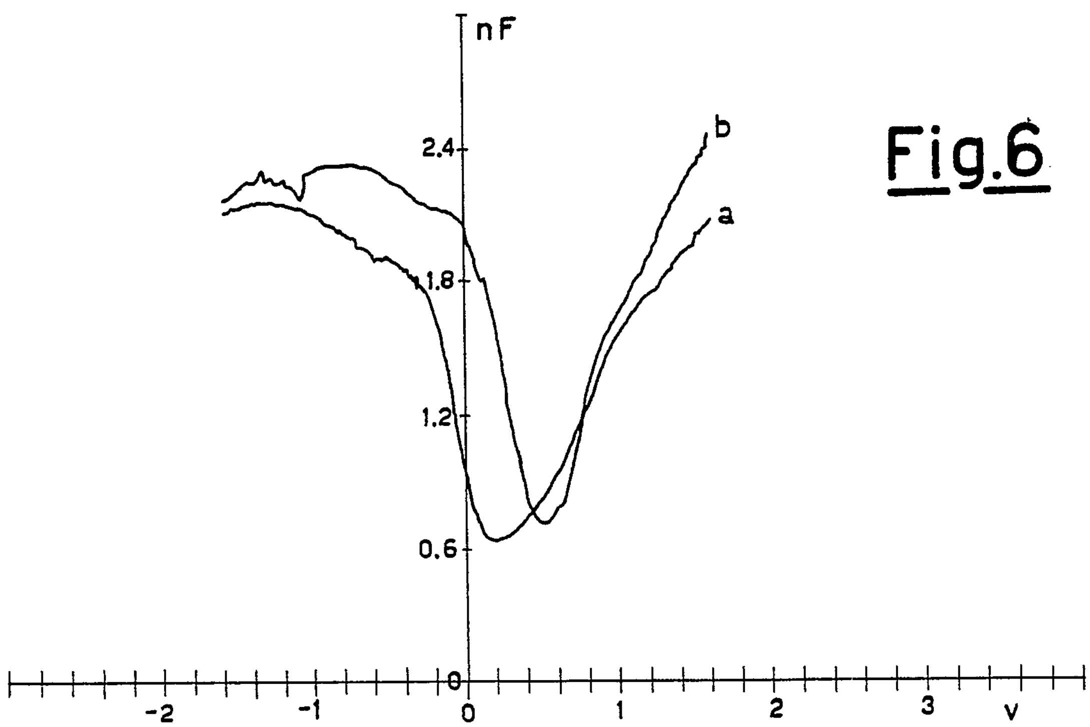
nF
2.4
1.8
1.2
0.6
0
b
a
-2
-1
0
1
2
3
V

Fig.6

Fig.7

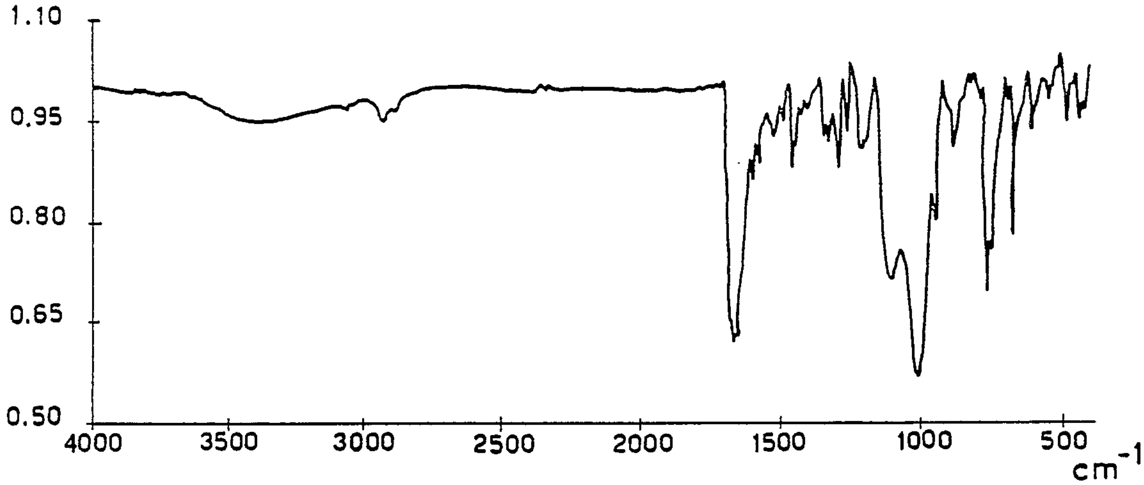


Fig.8

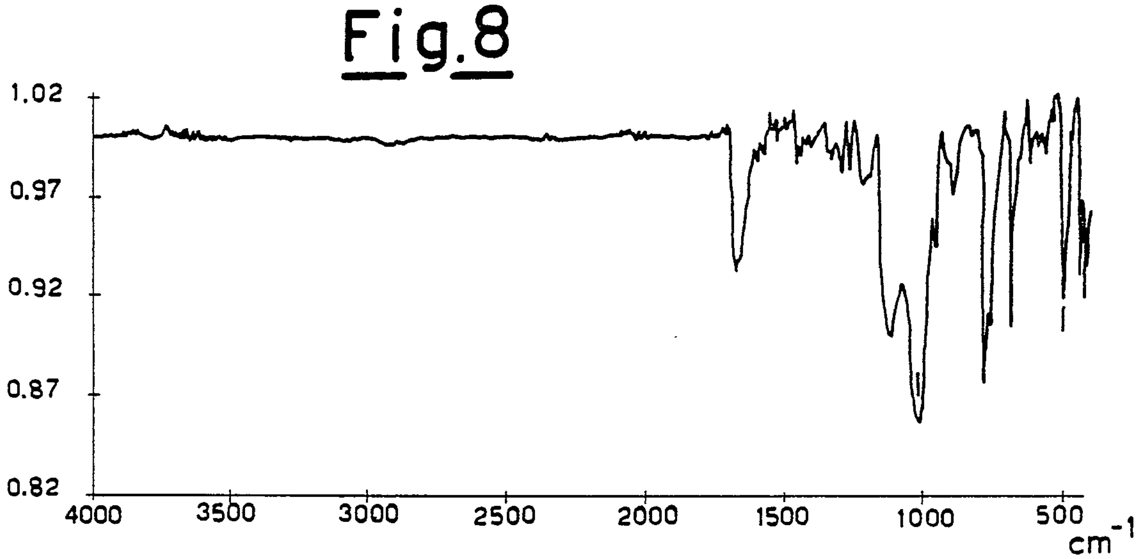


Fig.9

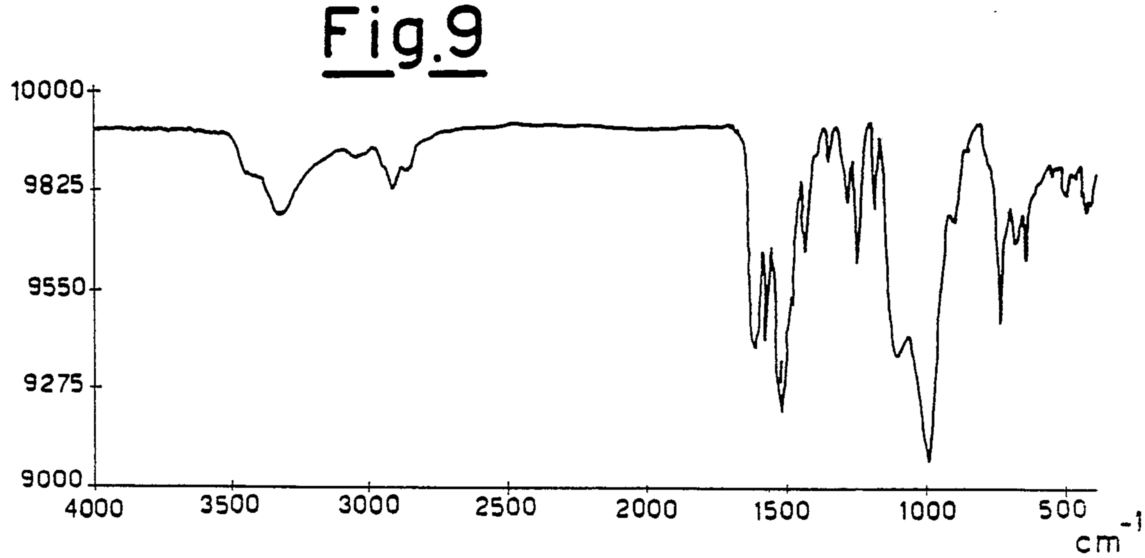

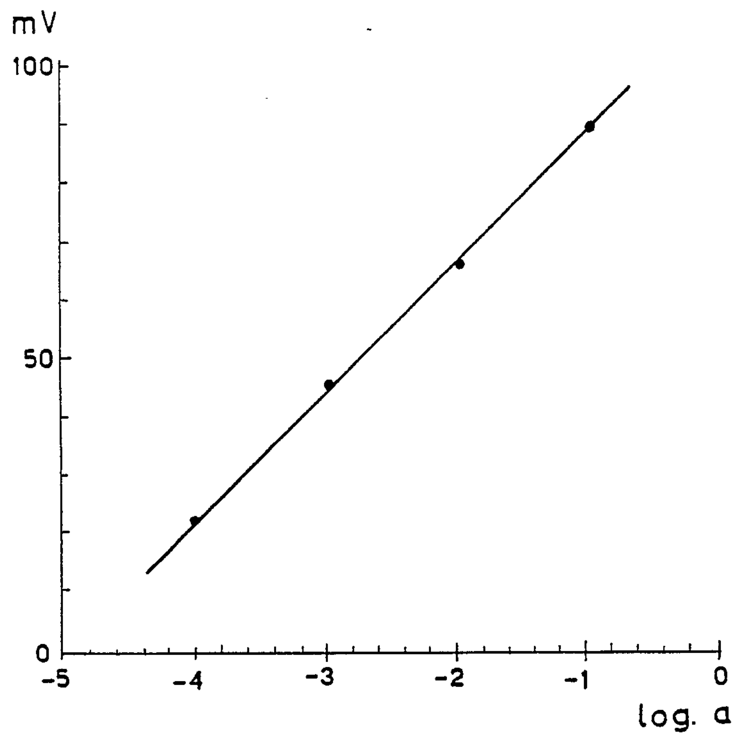
mV
100
50
0
-5
-4
-3
-2
-1
0
log. a

Fig.10

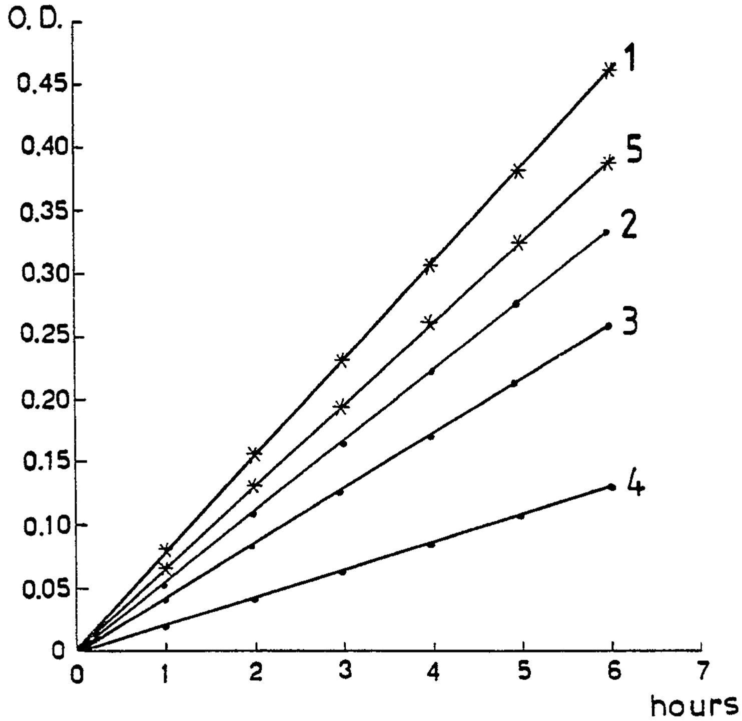
O.D.
0.45
0.40
0.35
0.30
0.25
0.20
0.15
0.10
0.05
0
0
1
2
3
4
5
6
7
hours
1
5
2
3
4

Fig.11